# EUROPEAN PATENT APPLICATION

(11) **EP 4 428 151 A1**
(43) Date of publication of application: **11.09.2024**
(21) Application number: 22900296.9
(22) Date of filing: 18.11.2022
(51) Int. Cl.: C07K 16/24, C12N 15/63, C12N 15/11, C12N 5/07, A61K 39/395, A61P 35/00, A61P 37/00

(54) **ANTI-TSLP MONOCLONAL ANTIBODY, ANTIGEN-BINDING FRAGMENT THEREOF AND USE THEREOF**

(30) Priority: 02.12.2021 CN 202111461974
(71) Applicant: Beijing Dongfang Biotech Co. Ltd., Beijing 100176 (CN)
(72) Inventor: BAI, Yi, Beijing 100176 (CN); PEI, Shuang, Beijing 100176 (CN); LIU, Si, Beijing 100176 (CN)
(74) Representative: Studio Torta S.p.A.
(86) International application number: PCT/CN2022/132848
(87) International publication number: WO 2023/098491

(57) **Abstract**

Provided are an anti-TSLP monoclonal antibody, an antigen-binding fragment thereof and a use thereof. The antibody has relatively high affinity with a TSLP antigen, may effectively inhibit the binding of the TSLP antigen to a receptor complex thereof, and then prevents a TSLP-targeted immune cell from releasing a pro-inflammatory cytokine, thereby asthma attack is prevented and asthma control is improved. The monoclonal antibody molecule obtained by screening in the present application also has relatively high thermal stability and relatively good safety. The present application may be used for treating asthma, chronic obstructive pulmonary disease, chronic eosinophilic pneumonia, idiopathic pulmonary fibrosis and allergic dermatitis; and the asthma includes severe asthma, eosinophilic or non-eosinophilic asthma, and low eosinophilic asthma.

## Description

### Cross-Reference to Related Application

The present application claims priority to Chinese patent application No. 202111461974.6 filed on December 02, 2021, the invention of which is hereby incorporated by reference in its entirety.

### Technical Field

The present invention relates to the technical field of biomedicines, and particularly relates to an anti-TSLP monoclonal antibody, an antigen-binding fragment thereof and a use thereof.

### Background

Asthma is one of the most common chronic diseases in the world at present, the incidence rate of the asthma is about 4.3%, there are about 300 million asthma patients in the world, and the prevalence rates of the asthma varies among countries from 1% to 18%, herein 10-20% of severe asthma patients are very difficult to control with current treatment methods, and these patients consume 50-60% of medical expenses for the asthma. Due to the constraints of economic conditions, in the vast developing countries, including China, many patients have not received effective diagnosis and treatment. On June 21, 2019, the international authoritative medical journal "The Lancet" published another important achievement of the large-scale population study Chinese Adult Pulmonary Health Study" (CPH Study) completed by Chinese scholars, which revealed the prevalence status of the asthma in China and confirmed that the prevalence rate of the asthma in the population aged 20 and above in China was 4.2%, and the number of cases reached 45.7 million. In China, asthma has already become one of the major public health and healthcare issues that need to be faced and addressed seriously. The Chinese Guidelines for Prevention and Treatment of Bronchial Asthma indicate that more than 70% of domestic asthma patients still have poor control, the emergency and hospitalization rates of the patients reach 27% and 23% due to asthma attacks within a year, and the poor asthma control may seriously affect the quality of life of the patients.

Asthma is a chronic inflammatory disease of airways that involves a variety of inflammatory cells and mediators. This chronic inflammation is associated with airway hyperresponsiveness, and is clinically manifested by recurrent wheezing, shortness of breath, chest tightness, and cough, which often occurs and worsen at night and/or in the morning, and most patients may alleviate these symptoms either spontaneously or by treatment; and pathologically, it manifests as chronic inflammatory changes in the airways, as well as airway remodeling, the latter includes thickening of the airway wall, subcutaneous fibrosis due to the deposition of matrix and collagen, smooth muscle proliferation and hypertrophy, proliferation of myofibroblasts and mucosal gland, goblet cell metaplasia and proliferation, which brings great difficulties to the treatment. There are many types of asthma medications on the global market so far, most of which are chemical drugs. With the continuous development of biopharmaceuticals, the research and development of the biopharmaceuticals for the asthma increasingly attracts people's attention.

T2 inflammation driven (T2 high) asthma is present in more than two-thirds of the severe asthma patients, it is characterized by elevated levels of T2 inflammatory biomarkers. The identification of the diagnostic and predictive biomarkers (including blood eosinophil, serum IgE, fractional exhaled nitric oxide (FeNO), Periostin and the like) revolutionize the field of targeted treatment for the severe asthma. Monoclonal antibodies targeting Th2 driven inflammation are usually safe in adult patients with moderate and severe asthma. About one-third of the severe asthma patients do not have activated T2 inflammation pathway features, and the symptoms of non-T2 driven diseases in these patients are still uncontrolled when treated with clinical standard guidelines. The research on non-Type-2 asthma is not yet clear, and the further research is needed to determine the biomarkers to guide the targeted treatment of different forms of the non-Type-2 asthma.

Thymic stromal lymphopoietin (TSLP) is an epithelial cytokine generated against pro-inflammatory stimuli (such as allergens, viruses, and other pathogens in lungs), which has the effects of enhancing thymocyte cell proliferation. TSLP drives the release of downstream T2 cytokines, including IL-4, IL-5, and IL-13, it causes inflammation and asthma symptoms. TSLP may also activate various types of cells involved in non-T2 driven inflammation. Therefore, the early upstream activity of TSLP in inflammatory cascade reactions has already been identified as a potential target in a wide population of the asthma patients.

In addition, TSLP regulates immunity by activating immature dendritic cells (DC), lymphocytes, mast cells, basophiles, and eosinophils. TSLP initiates intracellular signal transduction by a complex formed by its specific receptor TSLPR and co-receptor IL-7Rα. TSLP firstly binds to TSLPR with high affinity, and then forms a ternary complex with an extracellular domain of IL-7Rα. Two opposite faces of TSLP respectively interact with TSLPR and IL-7Rα. The activation of STAT5 is a necessary signal for a TSLP mediated Th2 reaction. Anti-TSLP humanized monoclonal antibodies may specifically bind to human TSLP and block its interaction with the receptor complex, which may prevent TSLP targeted immune cells from releasing pro-inflammatory cytokines, thereby the asthma attack is prevented and the asthma control is improved.

At present, AstraZeneca and its partner Amgen's Tezepelumab (also known as AMG157) is a first TSLP targeted monoclonal antibody drug, but it has not marketed yet. It was shown from clinical research that the anti-TSLP humanized monoclonal antibodies act on the early upstream of the inflammatory cascade reactions and are suitable for a wide range of severe uncontrolled asthma patients, including non-T2 driven asthma patients. A concept validated inhaled allergen challenge study conducted in mild and atopic asthma patients proved that it may inhibit early and late asthma responses and reduce the T2 inflammatory biomarker levels. In view of the importance of TSLP targeted drugs in the treatment of asthma, there is an urgent need to develop monoclonal antibody therapy drugs with individual or adjuvant therapy for the asthma, to meet the needs of the asthma patients both domestically and internationally.

### Summary

In order to meet market demands, the present invention obtains an anti-TSLP monoclonal antibody, an antigen-binding fragment thereof and a use thereof that may specifically bind to TSLP and has relatively high biological activity by screening of an immune library.

Specific technical schemes of the present invention are as follows.

The present invention provides an anti-TSLP monoclonal antibody or an antigen-binding fragment thereof, comprising a heavy chain variable region and a light chain variable region, wherein the heavy chain variable region comprises three heavy chain complementarity-determining regions represented by HCDR1, HCDR2, and HCDR3 respectively, the light chain variable region comprises three light chain complementarity-determining regions represented by LCDR1, LCDR2, and LCDR3 respectively, and the monoclonal antibody or the antigen-binding fragment thereof is selected from any one of the following:
A-I: the heavy chain complementarity-determining region HCDR1 comprises an amino acid sequence as shown in SEQ ID NO: 1, the heavy chain complementarity-determining region HCDR2 comprises an amino acid sequence as shown in SEQ ID NO: 2, the heavy chain complementarity-determining region HCDR3 comprises an amino acid sequence as shown in SEQ ID NO: 3, the light chain complementarity-determining region LCDR1 comprises an amino acid sequence as shown in SEQ ID NO: 4, the light chain complementarity-determining region LCDR2 comprises an amino acid sequence as shown in SEQ ID NO: 5, and the light chain complementarity-determining region LCDR3 comprises an amino acid sequence as shown in SEQ ID NO: 6;
A-II: the heavy chain complementarity-determining region HCDR1 comprises an amino acid sequence as shown in SEQ ID NO: 7, the heavy chain complementarity-determining region HCDR2 comprises an amino acid sequence as shown in SEQ ID NO: 8, the heavy chain complementarity-determining region HCDR3 comprises an amino acid sequence as shown in SEQ ID NO: 9, the light chain complementarity-determining region LCDR1 comprises an amino acid sequence as shown in SEQ ID NO: 4, the light chain complementarity-determining region LCDR2 comprises an amino acid sequence as shown in SEQ ID NO: 10, and the light chain complementarity-determining region LCDR3 comprises an amino acid sequence as shown in SEQ ID NO: 6;
A-III: the heavy chain complementarity-determining region HCDR1 comprises an amino acid sequence as shown in SEQ ID NO: 1, the heavy chain complementarity-determining region HCDR2 comprises an amino acid sequence as shown in SEQ ID NO: 11, the heavy chain complementarity-determining region HCDR3 comprises an amino acid sequence as shown in SEQ ID NO: 3, the light chain complementarity-determining region LCDR1 comprises an amino acid sequence as shown in SEQ ID NO: 12, the light chain complementarity-determining region LCDR2 comprises an amino acid sequence as shown in SEQ ID NO: 13, and the light chain complementarity-determining region LCDR3 comprises an amino acid sequence as shown in SEQ ID NO: 14; and
A-IV: the heavy chain complementarity-determining region HCDR1 comprises an amino acid sequence as shown in SEQ ID NO: 1, the heavy chain complementarity-determining region HCDR2 comprises an amino acid sequence as shown in SEQ ID NO: 11, the heavy chain complementarity-determining region HCDR3 comprises an amino acid sequence as shown in SEQ ID NO: 3, the light chain complementarity-determining region LCDR1 comprises an amino acid sequence as shown in SEQ ID NO: 12, the light chain complementarity-determining region LCDR2 comprises an amino acid sequence as shown in SEQ ID NO: 15, and the light chain complementarity-determining region LCDR3 comprises an amino acid sequence as shown in SEQ ID NO: 16.

The present invention obtains the above 4 monoclonal antibody molecules that may bind to the TSLP antigen with high affinity by screening of the immune library, and have the good binding activity, thereby its interaction with a receptor complex is blocked, then a TSLP-targeted immune cell is prevented from releasing a pro-inflammatory cytokine, asthma attack is prevented and asthma control is improved. In addition, the monoclonal antibody molecules screened by the present invention also have the relatively high thermal stability and meet the conditions for pharmaceutical development.

Further, the anti-TSLP monoclonal antibody or the antigen-binding fragment thereof is a murine antibody molecule, and the murine antibody molecule is selected from any one of the following:
MA-I: the heavy chain variable region comprises an amino acid sequence as shown in SEQ ID NO: 17, and the light chain variable region comprises an amino acid sequence as shown in SEQ ID NO: 18;
MA-II: the heavy chain variable region comprises an amino acid sequence as shown in SEQ ID NO: 19, and the light chain variable region comprises an amino acid sequence as shown in SEQ ID NO: 20;
MA-III: the heavy chain variable region comprises an amino acid sequence as shown in SEQ ID NO: 21, and the light chain variable region comprises an amino acid sequence as shown in SEQ ID NO: 22; and
MA-IV: the heavy chain variable region comprises an amino acid sequence as shown in SEQ ID NO: 23, and the light chain variable region comprises an amino acid sequence as shown in SEQ ID NO: 24;
preferably, the murine antibody molecule is MA-I.

The present invention immunizes mice with the TSLP antigen, optimizes the immune method, creates the phage display library, and screens out the above murine antibody molecules with relatively high affinity, good activity and stability. By extensive cell level experiments, it is found that compared to the other three murine antibody molecules, MA-I has the higher biological activity. Therefore, the present invention preferably selects MA-I.

Further, the murine antibody molecule further comprises a murine antibody heavy chain constant region and a murine antibody light chain constant region, the murine antibody heavy chain constant region is selected from a heavy chain constant region of a mouse IgG1-type, IgG2a-type, IgG2b-type, or IgG3-type, wherein the amino acid sequence of the IgG1-type heavy chain constant region is shown in SEQ ID NO: 26, the amino acid sequence of the IgG2a-type heavy chain constant region is shown in SEQ ID NO: 27, the amino acid sequence of the IgG2b-type heavy chain constant region is shown in SEQ ID NO: 28, and the amino acid sequence of the IgG3-type heavy chain constant region is shown in SEQ ID NO: 29; the murine antibody light chain constant region is a mouse Cₖ-type light chain constant region, and its amino acid sequence is shown in SEQ ID NO: 25; and
preferably, the murine antibody molecule comprises the mouse IgG1-type heavy chain constant region and the mouse Cₖ-type light chain constant region.

Further, the monoclonal antibody or the antigen-binding fragment thereof is a chimeric antibody molecule, the chimeric antibody molecule comprises a heavy chain variable region of the murine antibody molecule, a light chain variable region of the murine antibody molecule, and a humanized antibody constant region.

The chimeric antibody molecule includes a variable region sequence of the murine antibody molecule and the humanized antibody constant region, and the design of the chimeric antibody molecule is used to verify that the humanization of the constant region of the present invention does not change the specific functions of complementarity-determining regions (CDR) and provide further research and development basis for the humanized antibody molecules.

Further, the monoclonal antibody or the antigen-binding fragment thereof is a humanized antibody molecule, and the humanized antibody molecule is selected from any one of the following:
HA-I: the heavy chain variable region comprises an amino acid sequence as shown in SEQ ID NO: 34, and the light chain variable region comprises an amino acid sequence as shown in SEQ ID NO: 35;
HA-II: the heavy chain variable region comprises an amino acid sequence as shown in SEQ ID NO: 34, and the light chain variable region comprises an amino acid sequence as shown in SEQ ID NO: 36;
HA-III: the heavy chain variable region comprises an amino acid sequence as shown in SEQ ID NO: 37, and the light chain variable region comprises an amino acid sequence as shown in SEQ ID NO: 38; and
HA-IV: the heavy chain variable region comprises an amino acid sequence as shown in SEQ ID NO: 37, and the light chain variable region comprises an amino acid sequence as shown in SEQ ID NO: 36;
preferably, the humanized antibody molecule is HA-I.

The present invention performs the humanized design on the murine antibody molecules and obtains the humanized antibody molecules after screening. By in vitro and in vivo experimental verification, it is found that among the 4 humanized antibody molecules provided by the present invention, HA-I has the highest biological activity and the most significant therapeutic effect. Therefore, the present invention preferably selects HA-I.

Further, the humanized antibody molecule further includes a humanized antibody constant region.

Further, the humanized antibody molecule is a full-length antibody or an antibody fragment, and the humanized antibody molecule includes one or a combination of Fab, F(ab)2, Fv, or ScFv.

Further, the humanized antibody constant region comprises a humanized antibody heavy chain constant region and a humanized antibody light chain constant region, the humanized antibody heavy chain constant region is selected from a heavy chain constant region of a human IgG1-type, IgG2-type, or IgG4-type, the amino acid sequence of the IgG1-type heavy chain constant region is shown in SEQ ID NO: 30, the amino acid sequence of the IgG2-type heavy chain constant region is shown in SEQ ID NO: 31, the amino acid sequence of the IgG4-type heavy chain constant region is shown in SEQ ID NO: 32, the humanized antibody light chain constant region is a human Cₖ-type light chain constant region, and its amino acid sequence is shown in SEQ ID NO: 33; and
preferably, the humanized antibody constant region comprises the human IgG1-type heavy chain constant region and the human Cₖ-type light chain constant region.

The present invention further provides a protein, and it comprises the anti-TSLP monoclonal antibody or the antigen-binding fragment thereof.

The present invention further provides a polynucleotide molecule, and the polynucleotide molecule encodes the anti-TSLP monoclonal antibody or the antigen-binding fragment thereof.

The present invention further provides a recombinant DNA expression vector, and the recombinant DNA expression vector comprises the polynucleotide molecule.

The present invention further provides a host cell transfected with the recombinant DNA expression vector, and the host cell includes a prokaryotic cell, a yeast cell, an insect cell, or a mammalian cell; and
preferably, the host cell is the mammalian cell, and the mammalian cell is a HEK293 cell, a CHO cell, or a NS0 cell.

The present invention further provides a drug, and the drug comprises the anti-TSLP monoclonal antibody or the antigen-binding fragment thereof.

The present invention further provides a use of the anti-TSLP monoclonal antibody or the antigen-binding fragment thereof in preparation of a drug for treating an immunological disease or a cancer;
preferably, the immunological disease comprises asthma, chronic obstructive pulmonary disease, chronic eosinophilic pneumonia, idiopathic pulmonary fibrosis, and allergic dermatitis; the asthma comprises severe asthma, eosinophilic or non-eosinophilic asthma, and low eosinophilic asthma; and
preferably, the cancer comprises pancreatic cancer, non-small cell lung cancer, melanoma, prostate cancer, kidney cancer, colorectal cancer, or breast cancer.

The present invention further provides a method for treating or preventing a TSLP mediated disease, and the method includes administering a therapeutically effective amount of the anti-TSLP monoclonal antibody to an individual in need, and the disease includes an immunological disease or a cancer;
the immunological disease includes asthma, chronic obstructive pulmonary disease, chronic eosinophilic pneumonia, idiopathic pulmonary fibrosis, and allergic dermatitis; the asthma includes severe asthma, eosinophilic or non-eosinophilic asthma, and low eosinophilic asthma; and
the cancer includes pancreatic cancer, non-small cell lung cancer, melanoma, prostate cancer, kidney cancer, colorectal cancer, or breast cancer.

The beneficial effects of the present invention at least include: the anti-TSLP monoclonal antibody or the antigen-binding fragment thereof provided by the present invention has the relatively high affinity with the TSLP antigen, and may effectively inhibit the binding of the TSLP antigen and its receptor complex, thereby the TSLP-targeted immune cell is prevented from releasing the pro-inflammatory cytokine, and the asthma attack is prevented and the asthma control is improved. In addition, the monoclonal antibody molecules screened by the present invention also have the relatively high thermal stability and good safety. The anti-TSLP monoclonal antibody or the antigen-binding fragment thereof screened by the present invention may be used for treating the immunological disease or the cancer, and the immunological disease includes but not limited to asthma, chronic obstructive pulmonary disease, chronic eosinophilic pneumonia, idiopathic pulmonary fibrosis, and allergic dermatitis; the asthma includes but not limited to severe asthma, eosinophilic or non-eosinophilic asthma, and low eosinophilic asthma; and the cancer includes but not limited to pancreatic cancer, non-small cell lung cancer, melanoma, prostate cancer, kidney cancer, colorectal cancer, or breast cancer.

### Brief Description of the Drawings

Fig. 1 is a plasmid profile of a pScFv-Disb-HS vector in example 2 of the present invention;
Fig. 2 is a comparison diagram of relative affinity of gradient diluted ELISA anti-TSLP phage monoclonal antibodies in example 3 of the present invention;
Fig. 3 is a spectrum of a vector pTSE in Example 5 of the present invention;
Fig. 4 is a denatured polyacrylamide gel electrophoresis diagram of a murine antibody molecule in Example 5 of the present invention;
Fig. 5 is a comparison diagram of binding ability between a murine antibody and TSLP in Example 6 of the present invention;
Fig. 6 is a comparison diagram of a competitive inhibition experiment between the murine antibody and a TSLP receptor protein CRLF2 in Example 7 of the present invention;
Fig. 7 is a denatured polyacrylamide gel electrophoresis diagram of a humanized antibody molecule in Example 12 of the present invention;
Fig. 8 is a comparison diagram of binding ability between the humanized antibody molecule and TSLP in Example 15 of the present invention;
Fig. 9 is a comparison diagram of the competitive inhibition experiment between a humanized antibody and a control antibody in Example 16 of the present invention;
Fig. 10 is a cross binding experiment diagram between the humanized antibody and different species of TSLP in Example 17 of the present invention;
Fig. 11 is a comparison diagram of an experiment in which an anti-TSLP monoclonal antibody inhibits the binding of TSLP to a cell surface receptor in Example 18 of the present invention;
Fig. 12 is a comparison diagram of biological activity detection (reporter gene method) of the anti-TSLP monoclonal antibody in Example 19 of the present invention;
Fig. 13 is a comparison diagram of the anti-TSLP monoclonal antibody blocking TSLP induced release of a chemokine in an mDC cell in Example 20 of the present invention; and
Fig. 14 is a thermal stability evaluation diagram of the anti-TSLP monoclonal antibody HA-1 in Example 21 of the present invention.

### Detailed Description of the Examples

In order to understand the present invention more easily, before examples are described, some technical and scientific terms of the present invention are firstly explained as follows.

The term "antibody" used in this article includes all antibodies and any antigen-binding fragments, the antibody includes a murine antibody, a humanized antibody, a bispecific antibody, or a chimeric antibody, the antibody may also be Fab, F(ab)2, Fv, or ScFv (single chain antibody), and the antibody may be an antibody existing naturally or an antibody modified (such as mutation, deletion, or substitution).

The terms "variable region" and "constant region" used in this article mean that sequence regions of antibody heavy chain and light chain near the N terminal are the variable region (V region), and the remaining amino acid sequences near the C terminal are relatively stable and are the constant region (C region). The variable region includes 3 complementarity-determining regions (CDR) and 4 frame regions (FR), each light chain variable region and heavy chain variable region both consists of 3 CDRs and 4 FRs, the 3 CDRs of the heavy chain are represented by HCDR1, HCDR2, and HCDR3 respectively, and the 3 CDRs of the light chain are represented by LCDR1, LCDR2, and LCDR3 respectively.

The term "murine antibody molecule" used in this article is derived from an antibody obtained by immunizing mice with a TSLP antigen.

The term "chimeric antibody molecule" used in this article refers to an antibody formed by fusing the variable region of the murine antibody with the constant region of the humanized antibody, which may alleviate the immune response induced by the murine antibody in the human body. The chimeric antibody is created using a DNA recombinant technology to insert variable region genes of the light and heavy chains of the murine monoclonal antibody into an expression vector containing the constant region of a humanized antibody. In this way, the variable region of the light and heavy chains in the expressed antibody molecule is murine, while the constant region is human-derived, and nearly two-thirds of the entire antibody molecule is human-derived. The antibody generated in this way reduces the immunogenicity of the murine antibody while retains the parental antibody's ability to specifically bind to the antigen.

The term "humanized antibody molecule" used in this article means that CDR of the murine monoclonal antibody is transplanted to the variable region of the humanized antibody, to replace CDR of the humanized antibody, so that the humanized antibody obtains the antigen-binding specificity of the murine monoclonal antibody and meanwhile the heterogeneity is reduced.

The term "CHO cell" refers to a Chinese hamster ovary cell, the term "HEK293 cell" refers to a human embryonic kidney 293 cell, and the term "NS0 cell" refers to a mouse NS0 thymoma cell.

The present invention is further described in detail below in combination with the examples.

### Example 1

Example 1 of the present invention provided an anti-TSLP monoclonal antibody or an antigen-binding fragment thereof, specifically including a heavy chain variable region and a light chain variable region, wherein the heavy chain variable region included 3 heavy chain complementarity-determining regions represented by HCDR1, HCDR2, and HCDR3 respectively, and the light chain variable region included 3 light chain complementarity-determining regions represented by LCDR1, LCDR2, and LCDR3 respectively. The monoclonal antibody or the antigen-binding fragment thereof was selected from any one of the following. The following CDRs were determined based on a Kabat numbering system.

| Combinat ion | HCDR 1 | HCDR2 | HCDR3 | LCDR1 | LCDR2 | LCDR3 |
|---|---|---|---|---|---|---|
| A-I | SYW MH | LIDPSDSDTTYNQ KFKG | SLDGYF DH | RASENIYSY LA | NARTL AE | QHHYGTP WT |
| | (SEQ ID NO: 1) | (SEQ ID NO: 2) | (SEQ ID NO: 3) | (SEQ ID NO: 4) | (SEQ ID NO: 5) | (SEQ ID NO: 6) |
| A-II | TYWM H | VIDPSDSYITYNQK FKG | SLDGYF DY | RASENIYSY LA | NAKTL AE | QHHYGTP WT |
| | (SEQ ID NO: 7) | (SEQ ID NO: 8) | (SEQ ID NO: 9) | (SEQ ID NO: 4) | (SEQ ID NO: 10) | (SEQ ID NO: 6) |
| A-III | SYW MH | VIDPSDSDTTYNQ KFKG | SLDGYF DH | RASGNIHN YLA | NAKTL AD | QHFWSTP WT |
| | (SEQ ID NO: 1) | (SEQ ID NO: 11) | (SEQ ID NO: 3) | (SEQ ID NO: 12) | (SEQ ID NO: 13) | (SEQ ID NO: 14) |
| A-IV | SYW MH | VIDPSDSDTTYNQ KFKG | SLDGYF DH | RASGNIHN YLA | NTKTL AD | QHFWSTP YT |
| | (SEQ ID NO: 1) | (SEQ ID NO: 11) | (SEQ ID NO: 3) | (SEQ ID NO: 12) | (SEQ ID NO: 15) | (SEQ ID NO: 16) |

### Example 2: Screening of murine antibody molecule

The present invention immunized mice with a TSLP antigen, optimized an immune method, created a phage display library, and established an antigen site screening method. The specific construction and screening identification of the phage display library were as follows.

### Step 1: Immunizing mice with TSLP antigen

### 1. Experimental animal:

Species and strain: BALB/c, female, and mouse;
Weight: 18-20 g;
Experimental animal provider: Beijing HFK Bioscience Co., Ltd.

2. Immunization: the mice were immunized with a human TSLP (a gene synthesized by Nanjing Genscript Biotechnology Co., Ltd., our company constructed a vector and expressed and purified it) as the immune antigen.

### Step 2: Construction of phage antibody library

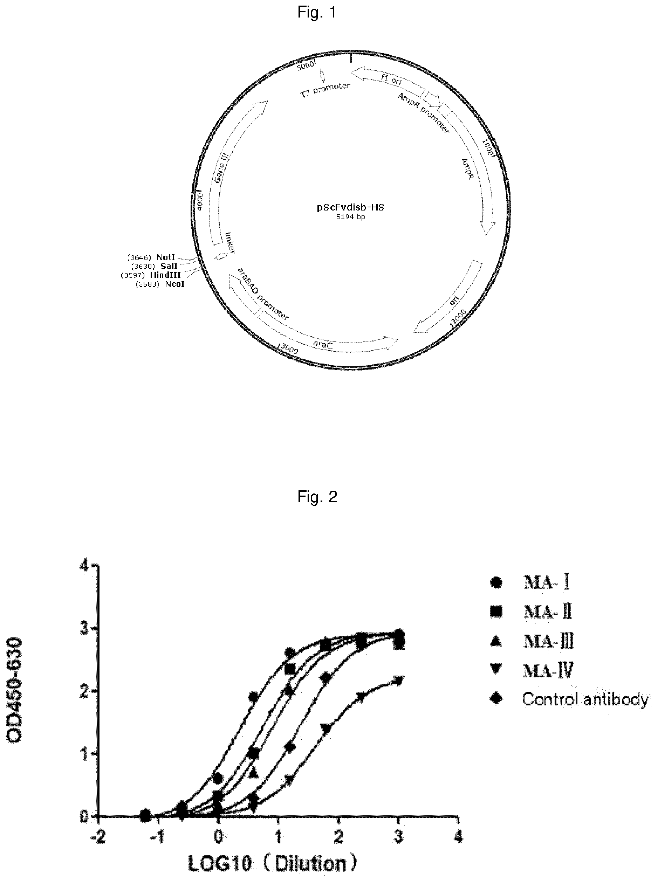

Mouse spleen cells with high potency were taken, a Trizol reagent (purchased from Ambion, product code: 15596026) was used to extract total RNA from the mouse spleen cells, cDNA was acquired by RT-PCR, cDNA was used as a template, a degenerate primer (the degenerate primer used referred to a document: Journal of Immunological Methods 233 (2000) 167-177) was used for polymerase chain reaction (PCR) amplification, and immune mouse antibody heavy chain variable region gene library (VH) and light chain variable region gene library (VL) were acquired. The light and heavy chains were double-digested and connected to a vector digested in the same step, to construct a pScFv-Disb-HS-VH-VL gene library, and the PscFv-DisB-HS vector was modified with a series of gene cloning methods, to construct and express a phage single chain antibody library using a pComb3 vector (purchased from the Biovector Science Lab). The modified vector was named as a pScFv-Disb-HS vector, and its plasmid profile was acquired as shown in Fig. 1. Based on this vector, the mouse immune phage antibody library was constructed.

Step 3: an immune tube was coated with TSLP as the antigen, the antigen coating amount was 5 µg/500 µl/tube, it was coated overnight at 4°C, and then sealed with 4% skim milk powder/PBST to respectively block the immune tube and immune phage antibody library, and sealed at a room temperature for 1 h. The sealed immune phage antibody library was added to the immune tube for antigen-antibody binding. The amount of phages added was about 10⁹∼10¹². After being reacted at the room temperature for 1 h, the unbound phages were washed off with PBST-PBS and eluted with Glycine-HCl at 0.1 M and pH 2.2. Finally, the eluted phage antibody solution was neutralized with Tris-HCl at 1.5 M and pH 8.8 to about pH 7.0.

Step 4: the above phages neutralized were infected with 10 ml of TG1 bacterial solution grown to a logarithmic phase, it was stilly placed in a 37°C incubator for 30 min, a part of the bacterial solution was taken for gradient dilution and applied on a 2YTAG plate for calculating the phage production. The remaining bacterial solution was centrifuged to discard a supernatant, a bacterial precipitate was resuspended on a small amount of a culture medium and applied on a 2YTAG large plate after being sucked, to prepare for the next round of screening.

Step 5: the above infected bacterial cells were scraped from the large plate, inoculated into a 2YTAG liquid culture medium, and grown to a logarithmic phase, M13KO7 was added to assist in phage superinfection. It was cultured overnight at 28°C and 220 rpm to prepare phages, and the phages were precipitated and purified with PEG/NaCl for the next round of screening. A total of one round of phage library enrichment screening was conducted.

Step 6: Screening of TSLP phage single chain antibody positive clones: after one round of screening, monoclonal colonies which were separated well were selected, inoculated on a 96-well deep well plate added with the 2YTAG liquid culture medium, and cultured at 37°C and 220 rpm until its logarithmic growth phase. About 10¹⁰ of auxiliary phage M13KO7 was added to each well and stilly infected for 30 min at 37°C. It was centrifuged at 4000 rpm for 15 min, a supernatant was discarded, bacterial cells were resuspended to precipitate, and cultured overnight at 28°C and 220 rpm. After being centrifuged at 4000 rpm and 4°C for 15 min, an amplified phage supernatant was sucked for ELISA identification. Finally, four anti-TSLP murine antibody molecules with relatively high affinity were obtained by screening, and named as MA-I, MA-II, MA-III, and MA-IV respectively. The above monoclonal antibodies obtained were genetically sequenced to determine the correct antibody sequences. After being sequenced, the sequences of the above 4 screened monoclonal antibody were as follows.

| Murine antibody molecule | Heavy chain variable region sequence | Light chain variable region sequence |
|---|---|---|
| MA-I | SEQ ID NO:17 | SEQ ID NO:18 |
| MA-II | SEQ ID NO:19 | SEQ ID NO:20 |
| MA-III | SEQ ID NO:21 | SEQ ID NO:22 |
| MA-IV | SEQ ID NO:23 | SEQ ID NO:24 |

Specifically, SEQ ID NO: 17 (the amino acid sequence of the heavy chain variable region of MA-I): 
QVQLEQSGAELVKPGASVKMSCKASGYTFTSYWMHWVKQRPGQGLEWIGLIDPSDSDTTY
NQKFKGKATLTVDTSSSTAYMQLGSLTSEDSAVYYCSRSLDGYFDHWGQGTLVTVSA;
SEQ ID NO: 18 (the amino acid sequence of the light chain variable region of MA-I): 
DIVMTQSPASLSASVGETVTITCRASENIYSYLAWYQQKQGKSPQLLVYNARTLAEGVPSRFS
GSGSGTQFSLKINSLQPEDFGSFYCQHHYGTPWTFGGGTKLEIK;
SEQ ID NO: 19 (the amino acid sequence of the heavy chain variable region of MA-II): 
QVKLQQSGAELVKPGASVKMSCKASGYTFTTYWMHWVKQRPGQGLEWIGVIDPSDSYITY
NQKFKGKATLTVDTSSSTAYMQLSSLTSEDSAVYYCTRSLDGYFDYWGQGTLVTVSA;
SEQ ID NO: 20 (the amino acid sequence of the light chain variable region of MA-II): 
DIVLTQSPASLSASVGETVTITCRASENIYSYLAWYQQKQGKSPQLLVYNAKTLAEGVPSRFS
GSGSGTQFSLKINSLQPEDFGSYYCQHHYGTPWTFGGGTKLEIK;
SEQ ID NO: 21 (the amino acid sequence of the heavy chain variable region of MA-III): 
QVQLQQPGAELVKPGASVKMSCKASGYTFTSYWMHWVKQRPGQGLEWIGVIDPSDSDTTY
NQKFKGKATLTVDTSSSTVYMQLSSLTSEDSAVYYCTRSLDGYFDHWGQGTLVTVSA;
SEQ ID NO: 22 (the amino acid sequence of the light chain variable region of MA-III): 
DIVMTQSPASLSASVGETVTITCRASGNIHNYLAWYQQKQGKSPQLLVYNAKTLADGVPSRF
SGSGSGTQYSLKINSLQPEDFGSYYCQHFWSTPWTFGGGTKLEIK;
SEQ ID NO: 23 (the amino acid sequence of the heavy chain variable region of MA-IV): 
QVKLEQSGAELVKPGASVKMSCKASGYTFTSYWMHWVKQRPGQGLEWIGVIDPSDSDTTY
NQKFKGKATLTVDTSSSTAYMQLSSLTSEDSAVYYCTRSLDGYFDHWGQGTLVTVSA;
SEQ ID NO: 24 (the amino acid sequence of the light chain variable region of MA-IV):

DIVITQTPASLSASVGETVTITCRASGNIHNYLAWYQQKQGKSPQLLVYNTKTLADGVPSRFS
GSGSGTQYSLKINSLQPEDFGSYYCQHFWSTPYTFGGGTKLEIK.

From another perspective, the present invention further related to an anti-TSLP monoclonal antibody or an antigen-binding fragment thereof, specifically including a heavy chain variable region and a light chain variable region, herein the heavy chain variable region included 3 heavy chain complementarity-determining regions represented by HCDR1, HCDR2, and HCDR3 respectively, and the light chain variable region included 3 light chain complementarity-determining regions represented by LCDR1, LCDR2, and LCDR3 respectively. Herein, HCDR1, HCDR2, and HCDR3 were HCDR1, HCDR2, and HCDR3 of the heavy chain variable region shown in SEQ ID NO: 17, 19, 21, or 23 respectively; and LCDR1, LCDR2, and LCDR3 were LCDR1, LCDR2, and LCDR3 of the light chain variable region shown in SEQ ID NO: 18, 20, 22, or 24 respectively. Those skilled in the art might determine the CDR sequences of the known heavy chain variable region or the light chain variable region of the amino acid sequence based on common numbering systems (such as Kabat, AbM, Chothia, Contact, or IMGT). When the Kabat numbering system was used to determine CDR, the CDR sequences of the heavy chain variable region and the light chain variable region were shown in Example 1.

### Example 3: Comparison of affinity of anti-TSLP phage monoclonal antibody using gradient dilution ELISA

Display and purification of monoclonal phages were performed on 4 murine antibody molecules (MA-I, MA-II, MA-III, MA-III, and MA-IV) obtained in Example 2, and then a phage gradient dilution ELISA experiment was performed to identify affinity. Amgen Company's anti-TSLP monoclonal antibody Tezepelumab (also known as AMG157, the patent application No. was CN201880026131.3, and the patent title was "Treatment of Asthma with Anti-TSLP Antibody") was selected as a control antibody, and a specific method was as follows.

TSLP was coated with pH 9.6 carbonate buffer solution at 100 ng/well/100 µl, coated overnight at a temperature of 4°C, and washed for three times with PBST. The 4 phage monoclonal antibodies screened in Example 2 were diluted respectively with PBST in a four-fold gradient, 100 µl of a diluted sample was added to each well, and it was stilly placed at a room temperature for 1 h. An ELISA plate was washed with PBST, and a HRP-anti-M13 (purchased from Bio-viewshine, product code: GE27-9421-01) monoclonal antibody diluted with PBST was added to the ELISA plate, and it was placed at the room temperature for 1 h. A TMB color kit was used for color development, it was color-developed at the room temperature for 10 min, and after being terminated with 2 M H₂SO₄, the ELISA plate was read at 450 nm/630 nm, and the corresponding EC50 value was calculated. Specific data was as follows.

| Clone | MA-I | MA-II | MA-III | MA-IV | Control antibody |
|---|---|---|---|---|---|
| EC50 | 2.28 | 5.515 | 8.038 | 41.38 | 23.14 |

Based on the above data and as shown in Fig. 2, the 4 different murine antibody molecules screened in Example 2 might all bind to TSLP. However, compared with the other 3 murine antibody molecules and the control antibody, the monoclonal antibody MA-I provided by the present invention had the higher affinity with TSLP.

### Example 4

On the basis of Example 2, Example 4 of the present invention further defined that the murine antibody molecule further included a murine antibody heavy chain constant region and a murine antibody light chain constant region, and the murine antibody heavy chain constant region was selected from a heavy chain constant region of a mouse IgG1-type, IgG2a-type, IgG2b-type, or IgG3-type; herein, the amino acid sequence of the IgG1-type heavy chain constant region was shown in SEQ ID NO: 26, the amino acid sequence of the IgG2a-type heavy chain constant region was shown in SEQ ID NO: 27, the amino acid sequence of the IgG2b-type heavy chain constant region was shown in SEQ ID NO: 28, and the amino acid sequence of the IgG3-type heavy chain constant region was shown in SEQ ID NO: 29; the light chain constant region of the murine antibody was a mouse Cₖ-type light chain constant region, and its amino acid sequence was shown in SEQ ID NO: 25; and the specific sequence was as follows.
SEQ ID NO: 25 (the amino acid sequence of the mouse Cₖ-type light chain constant region): 
ADAAPTVSIFPPSSEQLTSGGASVVCFLNNFYPKDINVKWKIDGSERQNGVLNSWTDQDSKD
STYSMSSTLTLTKDEYERHNSYTCEATHKTSTSPIVKSFNRNEC;
SEQ ID NO: 26 (the amino acid sequence of the mouse IgG1-type heavy chain constant region): 
AKTTPPSVYPLAPGSAAQTNSMVTLGCLVKGYFPEPVTVTWNSGSLSSGVHTFPAVLQSDLY
TLSSSVTVPSSTWPSETVTCNVAHPASSTKVDKKIVPRDCGCKPCICTVPEVSSVFIFPPKPKDVLTI
TLTPKVTCVVVDISKDDPEVQFSWFVDDVEVHTAQTQPREEQFNSTFRSVSELPIMHQDWLNGKE
FKCRVNSAAFPAPIEKTISKTKGRPKAPQVYTIPPPKEQMAKDKVSLTCMITDFFPEDITVEWQWN
GQPAENYKNTQPIMDTDGSYFVYSKLNVQKSNWEAGNTFTCSVLHEGLHNHHTEKSLSHSPG;
SEQ ID NO: 27 (the amino acid sequence of the mouse IgG2a-type heavy chain constant region): 
AKTTAPSVYPLAPVCGDTTGSSVTLGCLVKGYFPEPVTLTWNSGSLSSGVHTFPAVLQSDLY
TLSSSVTVTSSTWPSQSITCNVAHPASSTKVDKKIEPRGPTIKPCPPCKCPAPNLLGGPSVFIFPPKIK
DVLMISLSPIVTCVVVDVSEDDPDVQISWFVNNVEVHTAQTQTHREDYNSTLRVVSALPIQHQDW
MSGKEFKCKVNNKDLPAPIERTISKPKGSVRAPQVYVLPPPEEEMTKKQVTLTCMVTDFMPEDIY
VEWTNNGKTELNYKNTEPVLDSDGSYFMYSKLRVEKKNWVERNSYSCSVVHEGLHNHHTTKSF
SRTPGK;
SEQ ID NO: 28 (the amino acid sequence of the mouse IgG2b-type heavy chain constant region): 
AKTTPPSVYPLAPGCGDTTGSSVTLGCLVKGYFPESVTVTWNSGSLSSSVHTFPALLQSGLYT
MSSSVTVPSSTWPSQTVTCSVAHPASSTTVDKKLEPSGPISTINPCPPCKECHKCPAPNLEGGPSVFI
FPPNIKDVLMISLTPKVTCVVVDVSEDDPDVQISWFVNNVEVHTAQTQTHREDYNSTIRVVSTLPI
QHQDWMSGKEFKCKVNNKDLPSPIERTISKIKGLVRAPQVYILPPPAEQLSRKDVSLTCLVVGFNP
GDISVEWTSNGHTEENYKDTAPVLDSDGSYFIYSKLNMKTSKWEKTDSFSCNVRHEGLKNYYLK
KTISRSPGK;
SEQ ID NO: 29 (the amino acid sequence of the mouse IgG3-type heavy chain constant region):

ATTTAPSVYPLVPGCSDTSGSSVTLGCLVKGYFPEPVTVKWNYGALSSGVRTVSSVLQSGFY
SLSSLVTVPSSTWPSQTVICNVAHPASKTELIKRIEPRIPKPSTPPGSSCPPGNILGGPSVFIFPPKPKD
ALMISLTPKVTCVVVDVSEDDPDVHVSWFVDNKEVHTAWTQPREAQYNSTFRVVSALPIQHQD
WMRGKEFKCKVNNKALPAPIERTISKPKGRAQTPQVYTIPPPREQMSKKKVSLTCLVTNFFSEAIS
VEWERNGELEQDYKNTPPILDSDGTYFLYSKLTVDTDSWLQGEIFTCSVVHEALHNHHTQKNLSR
SPELELNETCAEAQDGELDGLWTTITIFISLFLLSVCYSASVTLFKVKWIFSSVVQVKQTAIPDYRN
MIGQGA.

### Example 5: Preparation of anti-TSLP murine antibody molecule

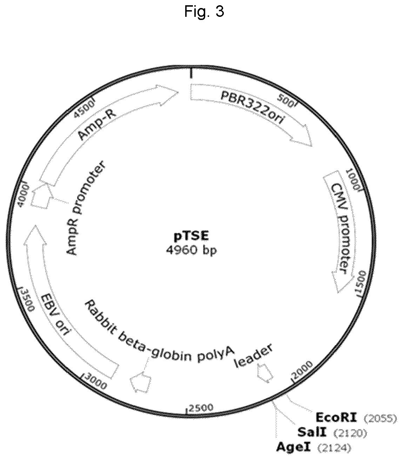

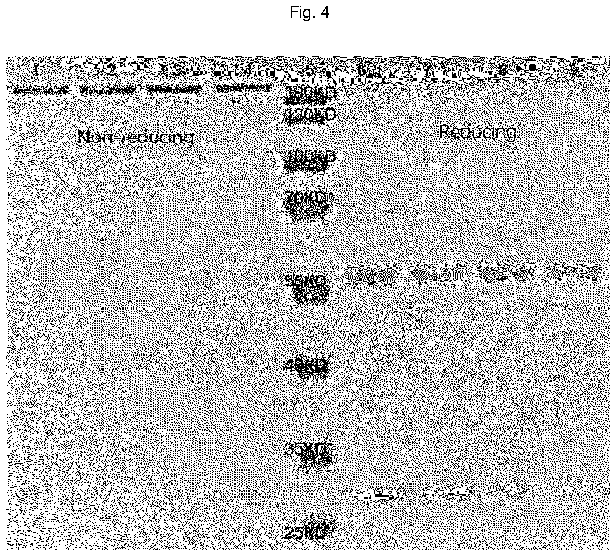

On the basis of Example 4, Example 5 of the present invention preferably defined that the murine antibody molecule included a mouse IgG1-type heavy chain constant region (its amino acid sequence was shown in SEQ ID NO: 26) and a mouse Cₖ-type light chain constant region (its amino acid sequence was shown in SEQ ID NO: 25). A preparation method for the antibody was specifically as follows.
1. Coding genes of the heavy chain VH and the light chain VL of the 4 monoclonal antibodies screened in Example 2 were cloned into a vector pTSE loaded with heavy chain and light chain constant region genes respectively (as shown in Fig. 3), preferably the heavy chain constant region was the mouse IgG1-type constant region (the amino acid sequence was shown in SEQ ID NO: 26), and the light chain constant region was the mouse Cₖ-type constant region (the amino acid sequence was shown in SEQ ID NO: 25). The structure of the pTSE vector was shown in Fig. 3 (a preparation process of the pTSE vector might be found in Paragraph [0019], Page 3 of the description in CN103525868A).
2. Transient transfection of HEK293E cells (purchased from the Institute of Basic Medicine, Chinese Academy of Medical Sciences, product code: GNHu43) was performed for antibody expression, and 4 monoclonal antibodies were obtained by protein A affinity column purification using an AKTA instrument. At the same time, the protein concentration was determined with a BCA kit (purchased from Beijing Huitian Dongfang Science and Technology Co., Ltd., product code: BCA0020), and the protein size was identified by SDS-PAGE. Results were shown in Fig. 4. From left to right, they were non-reducing MA-I, MA-II, MA-III, MA-III and MA-IV, as well as protein molecular weight Marker and reducing MA-I, MA-II, MA-III, MA-III and MA-IV murine anti-TSLP monoclonal antibodies sequentially, the molecular weight of each band was consistent with the theory.

### Example 6: Binding experiment between murine antibody and TSLP

TSLP was coated with pH 9.6 carbonate buffer solution at 100 ng/well/100 µl, and coated overnight at a temperature of 4°C. It was washed with 300 µl /well PBST for five times, 1% BSA-PBST was added, and it was sealed at 37°C for 1 h. Different dilution concentrations of MA-I, MA-II, MA-III, and MA-IV murine antibodies were added, the initial highest concentrations of all 4 antibodies were 1 µg/ml, it was diluted in a 5-fold gradient, and each antibody was diluted for a total of 8 gradients, and incubated at 37°C for 1 h. It was washed with 300 µl/well PBST for five times, then Goat Anti-Mouse IgG-HRP (purchased from solarbio, product code: SE131) diluted with 1% BSA-PBST 1:2000 was added and incubated at 37°C for 1 h. A TMB color kit was used for color development at 100 µl/well, it was color-developed at the room temperature for 8 min, then terminated with 2M H₂SO₄. An ELISA plate was read at 450 nm/630 nm, and the corresponding EC50 value was calculated. Specific data was as follows.

| Clone | MA-I | MA-II | MA-III | MA-IV |
|---|---|---|---|---|
| EC50(ng/ml) | 1.099 | 2.041 | 1.983 | 5.572 |

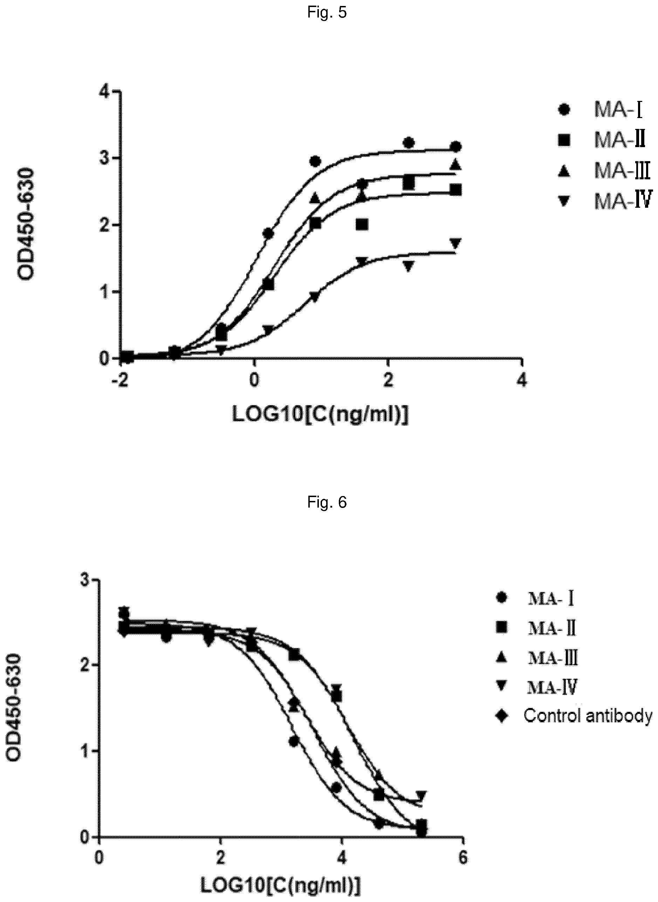

Based on the above data and as shown in Fig. 5, the 4 different murine antibodies screened might all bind to TSLP. In addition, among these 4 murine antibody molecules, the EC50 value of MA-I was the lowest, it was indicated that it had the better binding ability with TSLP.

### Example 7: Competitive inhibition experiment between murine antibody and TSLP receptor protein CRLF2

CRLF2-Fc was coated with pH 9.6 carbonate buffer solution at 200 ng/well/100 µl and coated overnight at a temperature of 4°C. It was washed with 300 µl /well PBST for five times, 1% BSA-PBST was added, and it was sealed at 37°C for 1 h. TSLP-His that was diluted to 10 µg/ml with 1% BSA-PBST was firstly added at 50 µl/well, then different dilution concentrations of MA-I, MA-II, MA-III, and MA-IV murine antibodies and a control antibody were added at 50 µl/well, the initial highest concentrations of all 5 antibodies were 400 µg/ml, it was diluted in a 5-fold gradient, and each antibody was diluted for a total of 8 gradients, and incubated at 37°C for 2 h. It was washed with 300 µl/well PBST for five times, then Anti-His-Tag Mouse-HRP (purchased from Beijing CWBio Technology Co., Ltd., product code: CW0285) diluted with 2% BSA-PBST 1:5000 was added and incubated at 37°C for 1 h. A TMB color kit was used for color development at 100 µl/well, it was color-developed at the room temperature for 10 min, then terminated with 2 M H₂SO₄. An ELISA plate was read at 450 nm/630 nm, and the corresponding EC50 value was calculated. Specific data was as follows.

| Clone | MA-I | MA-II | MA-III | MA-IV | Control antibody |
|---|---|---|---|---|---|
| IC50(ng/ml) | 1523 | 15626 | 2402 | 11816 | 3460 |

Based on the above data and as shown in Fig. 6, the 4 different murine antibodies screened might compete with the receptor protein CRLF2. In addition, among the 4 murine antibody molecules provided by the present invention, the IC50 value of MA-I was the lowest and significantly better than the control antibody, it was indicated that it might effectively inhibit the binding of TSLP to the receptor protein CRLF2.

### Example 8

Example 8 of the present invention further defined that the monoclonal antibody or the antigen-binding fragment thereof was a chimeric antibody molecule, and the chimeric antibody molecule included the heavy chain variable region of the murine antibody molecule, the light chain variable region of the murine antibody molecule, and the humanized antibody constant region in Example 2. The humanized antibody constant region included a humanized antibody heavy chain constant region and a humanized antibody light chain constant region, and the humanized antibody heavy chain constant region was selected from a heavy chain constant region of human IgG1-type, IgG2-type, or IgG4-type. The amino acid sequence of the IgG1-type heavy chain constant region was shown in SEQ ID NO: 30, the amino acid sequence of the IgG2-type heavy chain constant region was shown in SEQ ID NO: 31, the amino acid sequence of the IgG4-type heavy chain constant region was shown in SEQ ID NO: 32, the humanized antibody light chain constant region was a human Cₖ-type light chain constant region, and its amino acid sequence was shown in SEQ ID NO: 33.
SEQ ID NO: 30 (the amino acid sequence of the human IgG1-type heavy chain constant region): 
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEAAGGPSVFLFPPKP
KDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHEALHNHYTQKSL
SLSPGK;
SEQ ID NO: 31 (the amino acid sequence of the human IgG2-type heavy chain constant region): 
ASTKGPSVFPLAPCSRSTSESTAALGCLVKDYFPEPVTVSWNSGALTSGVHTFPAVLQSSGLY
SLSSVVTVPSSNFGTQTYTCNVDHKPSNTKVDKTVERKCCVECPPCPAPPVAGPSVFLFPPKPKDT
LMISRTPEVTCVVVDVSHEDPEVQFNWYVDGVEVHNAKTKPREEQFNSTFRVVSVLTVVHQDWL

NGKEYKCKVSNKGLPAPIEKTISKTKGQPREPQVYTLPPSREEMTKNQVSLTCLVKGFYPSDIAVE
WESNGQPENNYKTTPPMLDSDGSFFLYSKLTVDKSRWQQGNVFSCSVMHEALHNHYTQKSLSLS
PGK;
SEQ ID NO: 32 (the amino acid sequence of the human IgG4-type heavy chain constant region): 
ASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPPCPAPEFLGGPSVFLFPPKPKDT
LMISRTPEVTCVVVDVSQEDPEVQFNWYVDGVEVHNAKTKPREEQFNSTYRVVSVLTVLHQDW
LNGKEYKCKVSNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEALHNHYTQKSLSL
SLGK;
SEQ ID NO: 33 (the amino acid sequence of the human Cₖ-type light chain constant region):


.

### Example 9: Preparation of chimeric antibody molecule

On the basis of Example 8, Example 9 of the present invention further defined that the humanized antibody constant region included the human IgG1-type heavy chain constant region (its amino acid sequence was shown in SEQ ID NO: 30) and the human Cₖ-type light chain constant region (its amino acid sequence was shown in SEQ ID NO: 33).

### Specific preparation method:

Heavy chain variable region VH (SEQ ID NO: 17) and light chain variable region VL (SEQ ID NO: 18) genes of the antibody molecule MA-I screened from the immune phage antibody library in Example 2 were cloned on a vector pTSE loaded with the heavy chain constant region and light chain constant region genes (as shown in Fig. 3), the heavy chain constant region was the human IgG1-type (the amino acid sequence was shown in SEQ ID NO: 30) and the light chain constant region was the human Cₖ-type (the amino acid sequence was shown in SEQ ID NO: 33). Transient transfection of HEK293E cells (purchased from the Institute of Basic Medicine, Chinese Academy of Medical Sciences, product code: GNHu43) was performed for antibody expression, to obtain the chimeric antibody CA-I.

### Example 10: Humanization of murine antibody molecule MA-I

Firstly, the sequence of the murine antibody molecule MA-I in Example 2 was compared with a human antibody lineage database (v-base), to search for human antibody light and heavy chain lineages with relatively high homology as candidate sequences. Then, the CDR sequences of the murine antibody molecule MA-I were transplanted on the humanized candidate sequences for homology modeling. Then, key framework amino acid residues that might play an important role in maintaining the circular structure of CDR were calculated by a three-dimensional structural simulation, as to design a reverse mutation of the humanized antibody. The light and heavy chain variable regions of the designed humanized antibody containing the reverse mutation were optimized and synthesized by Nanjing Genscript Biotechnology Co., Ltd., and then connected to a transient expression vector. Humanized light and heavy chain combinations were analyzed, to obtain the following humanized antibody molecules: HA-I, HA-II, HA-III, and HA-IV. The 4 monoclonal antibody sequences screened above were as follows.

| Monoclonal antibody | Heavy chain variable region | Light chain variable region |
|---|---|---|
| HA-I | SEQ ID NO:34 | SEQ ID NO:35 |
| HA-II | SEQ ID NO:34 | SEQ ID NO:36 |
| HA-III | SEQ ID NO:37 | SEQ ID NO:38 |
| HA-IV | SEQ ID NO:37 | SEQ ID NO:36 |

Specifically, SEQ ID NO: 34 (the amino acid sequences of the heavy chain variable regions of HA-I and HA-II): 
QVQLVQSGAEVKKPGASVKVSCKASGYTFTSYWMHWVRQAPGQGLEWIGLIDPSDSDTTY
NQKFKGRATLTVDTSTSTAYMELSSLRSEDTAVYYCSRSLDGYFDHWGQGTLVTVSS;
SEQ ID NO: 35 (the amino acid sequence of the light chain variable region of HA-I): 
DIVMTQSPSSLSASVGDRVTITCRASENIYSYLAWYQQKPGKSPKLLVYNARTLAEGVPSRFS
GSGSGTDFTLTISSLQPEDFATYYCQHHYGTPWTFGGGTKVEIK;
SEQ ID NO: 36 (the amino acid sequences of the light chain variable regions of HA-II and HA-IV): 
DIVMTQSPSSLSASVGDRVTITCRASENIYSYLAWYQQKPGKAPKLLVYNARTLAEGVPSRFS
GSGSGTDFTLTISSLQPEDFATYYCQHHYGTPWTFGGGTKVEIK;
SEQ ID NO: 37 (the amino acid sequences of the heavy chain variable regions of HA-III and HA-IV): 
QVQLVQSGAEVKKPGASVKVSCKASGYTFTSYWMHWVRQAPGQGLEWIGLIDPSDSDTTY
NQKFKGRATMTVDTSTSTVYMELSSLRSEDTAVYYCARSLDGYFDHWGQGTLVTVSS;
SEQ ID NO: 38 (the amino acid sequence of the light chain variable region of HA-III):

DIQMTQSPSSLSASVGDRVTITCRASENIYSYLAWYQQKPGKAPKLLVYNARTLAEGVPSRFS
GSGSGTDFTLTISSLQPEDFATYYCQHHYGTPWTFGGGTKVEIK.

### Example 11

On the basis of Example 10, Example 11 of the present invention further defined that the humanized antibody molecule further included a humanized antibody constant region; and the humanized antibody constant region included a humanized antibody heavy chain constant region and a humanized antibody light chain constant region, the humanized antibody heavy chain constant region was selected from a heavy chain constant region of human IgG1-type, IgG2-type, or IgG4-type, the amino acid sequence of the IgG1-type heavy chain constant region was shown in SEQ ID NO: 30, the amino acid sequence of the IgG2-type heavy chain constant region was shown in SEQ ID NO: 31, the amino acid sequence of the IgG4-type heavy chain constant region was shown in SEQ ID NO: 32, the humanized antibody light chain constant region is a human Cₖ-type light chain constant region, and its amino acid sequence was shown in SEQ ID NO: 33.

The specific sequence of the above humanized antibody constant region was the same as Example 8.

### Example 12: Preparation of humanized antibody molecule

On the basis of Example 11, Example 12 of the present invention further defined that the humanized antibody constant region included a human IgG1-type heavy chain constant region (its amino acid sequence was shown in SEQ ID NO: 30) and a human Cₖ-type light chain constant region (its amino acid sequence was shown in SEQ ID NO: 33).

Coding genes of the heavy chain VH and the light chain VL of the 4 humanized antibody molecules obtained by humanization in the above Example 10 were cloned into a vector pTSE loaded with heavy chain and light chain constant region genes respectively (as shown in Fig. 3), the heavy chain constant region was the human IgG1-type (the amino acid sequence was shown in SEQ ID NO: 30), and the light chain constant region was the human Cₖ-type (the amino acid sequence was shown in SEQ ID NO: 33).

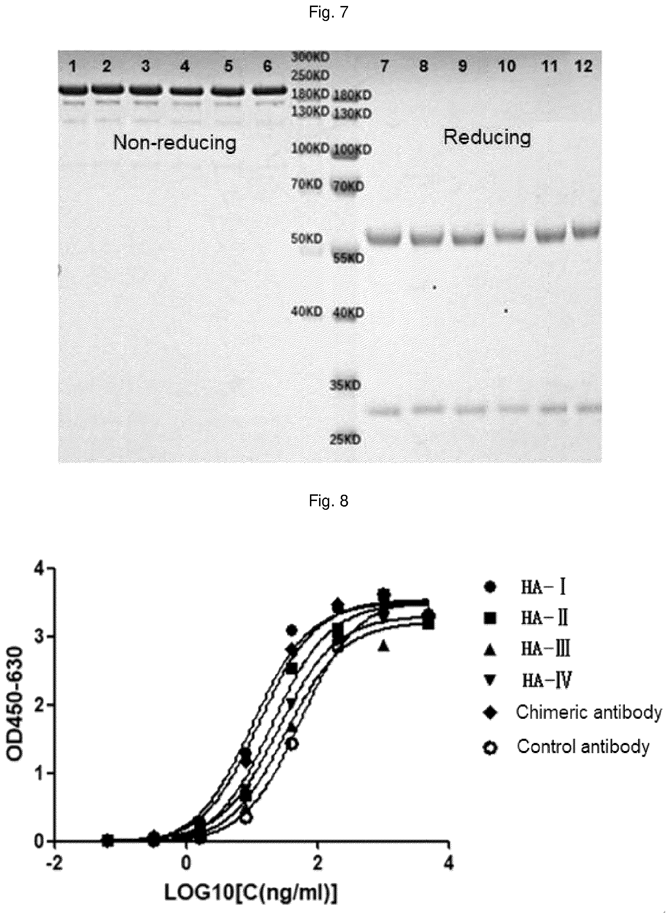

A control antibody and humanized antibody molecules HA-I, HA-II, HA-III, and HA-IV were transiently transfected into HEK293 cells (purchased from the Institute of Basic Medicine, Chinese Academy of Medical Sciences, product code: GNHu43) for antibody expression, and monoclonal antibodies were obtained by protein A affinity column purification using an AKTA instrument. At the same time, the protein concentration was determined with a BCA kit (purchased from Beijing Huitian Dongfang Science and Technology Co., Ltd., product code: BCA0020), and the protein size was identified by SDS-PAGE. Results were shown in Fig. 7. From left to right, they were non-reducing protein molecular weights Marker1, HA-I, HA-II, HA-III, and HA-IV, the chimeric antibody CA-I prepared in Example 9, and the control antibody, as well as reducing protein molecular weights Marker2, HA-I, HA-II, HA-III, and HA-IV, the chimeric antibody CA-I, and the control antibody sequentially, the molecular weight of each band was consistent with the theory.

### Example 13

On the basis of the above examples, Example 13 of the present invention further defined that the humanized antibody molecule was a full-length antibody or an antibody fragment, and the humanized antibody molecule included one or a combination of Fab, F(ab)2, Fv, or ScFv.

### Example 14

On the basis of the above examples, Example 14 of the present invention further defined the following schemes.

Further, the present invention further provided a protein, and it contained the anti-TSLP monoclonal antibody or the antigen-binding fragment thereof as defined in any one of the above examples.

The present invention further provided a polynucleotide molecule, and the polynucleotide molecule encoded the anti-TSLP monoclonal antibody or the antigen-binding fragment thereof as defined in any one of the above examples.

The present invention further provided a recombinant DNA expression vector, and the recombinant DNA expression vector contained the polynucleotide molecule defined above.

The present invention further provided a host cell transfected with the recombinant DNA expression vector defined above, and the host cell included a prokaryotic cell, a yeast cell, an insect cell, or a mammalian cell; and
preferably, the host cell was the mammalian cell, and the mammalian cell was a HEK293 cell, a CHO cell, or a NS0 cell.

The present invention further provided a drug, and the drug contained the anti-TSLP monoclonal antibody or the antigen-binding fragment thereof as defined in any one of the above examples.

The present invention further provided a use of the anti-TSLP monoclonal antibody or the antigen-binding fragment thereof in preparation of a drug for treating an immunological disease or a cancer.

The present invention provided a method for treating or preventing a TSLP mediated disease, the method included administering a therapeutically effective amount of the anti-TSLP monoclonal antibody to an individual in need, and the disease included an immunological disease or a cancer. Preferably, the immunological disease included but not limited to asthma, chronic obstructive pulmonary disease, chronic eosinophilic pneumonia, idiopathic pulmonary fibrosis, and allergic dermatitis; and the asthma included severe asthma, eosinophilic or non-eosinophilic asthma, and low eosinophilic asthma.

Preferably, the cancer included but not limited to pancreatic cancer, non-small cell lung cancer, melanoma, prostate cancer, kidney cancer, colorectal cancer, or breast cancer.

### Example 15: Binding experiment between humanized antibody molecule and TSLP

TSLP-His was coated with pH 9.6 carbonate buffer solution at 200 ng/well/100 µl, and coated overnight at a temperature of 4°C. It was washed with 300 µl /well PBST for five times, 1% BSA-PBST was added, and it was sealed at 37°C for 1 h. Different dilution concentrations of humanized antibodies HA-I, HA-II, HA-III, and HA-IV, as well as the chimeric antibody CA-I prepared in Example 9 and the control antibody were added, the initial highest concentrations of all 6 antibodies were 5 µg/ml, it was diluted in a 5-fold gradient, and each antibody was diluted for a total of 8 gradients, and incubated at 37°C for 1 h. It was washed with 300 µl/well PBST for five times, then Goat Anti-Human IgG-HRP (purchased from Beijing ZSGB Biotechnology Co., Ltd., product code: ZB-2304) diluted with 1% BSA-PBST 1 :5000 was added and incubated at 37°C for 1 h. A TMB color kit was used for color development at 100 µl/well, it was color-developed at the room temperature for 5 min, then terminated with 2M H₂SO₄. An ELISA plate was read at 450 nm/630 nm, and the corresponding EC50 value was calculated. Specific data was as follows.

| Clone | HA-I | HA-II | HA-III | HA-IV | Chimeric antibody CA-I | Control antibody |
|---|---|---|---|---|---|---|
| EC50(ng/ml) | 10.16 | 20.12 | 32.9 | 25.57 | 13.06 | 54.99 |

Based on the above data and experimental results as shown in Fig. 8, all 4 different humanized antibody molecules might bind to TSLP, and the EC50 values of the 4 different monoclonal antibodies provided by the present invention were significantly lower than that of the control antibody, it was indicated that the monoclonal antibody provided by the present invention had strong binding ability and high affinity with TSLP. In addition, from Fig. 8 and the above data, it might be concluded that among the 4 different monoclonal antibodies, the EC50 value of HA-I was the lowest, it was indicated that it had the best binding ability and highest affinity with TSLP; at the same time, the EC50 value of HA-I was similar to that of the chimeric antibody CA-I, it was indicated that the humanized HA-I retained the high affinity of the murine parent antibody MA-I with TSLP.

### Example 16: Competitive inhibition experiment between humanized antibody and control antibody

TSLP-His was coated with pH 9.6 carbonate buffer solution at 100 ng/well/100 µl and coated overnight at a temperature of 4°C. It was washed with 300 µl /well PBST for five times, 1% BSA-PBST was added, and it was sealed at 37°C for 1 h. HA-I, HA-II, HA-III, HA-IV and chimeric antibody CA-I that were diluted to 4 µg/ml with 1% BSA-PBST were firstly added at 50 µl/well, then different dilution concentrations of control antibodies were added at 50 µl/well, the initial highest concentrations of the control antibodies were 400 µg/ml, it was diluted in a 3-fold gradient, there was a total of 11 gradients, and it was incubated at 37°C for 2 h. It was washed with 300 µl/well PBST for five times, then Anti-Human IgG1-HRP (purchased from Sigma, product code: SAB 4200768) diluted with 1% BSA-PBST 1 :5000 was added and incubated at 37°C for 1 h. A TMB color kit was used for color development at 100 µl/well, it was color-developed at the room temperature for 10 min, then terminated with 2 M H₂SO₄. An ELISA plate was read at 450 nm/630 nm, and the corresponding EC50 value was calculated. Specific data was as follows.

| Clone | HA-I | HA-II | HA-III | HA-IV | Chimeric antibody CA-I |
|---|---|---|---|---|---|
| IC50(ng/ml) | 475.4 | 626.4 | 1633 | 977.7 | 627.3 |

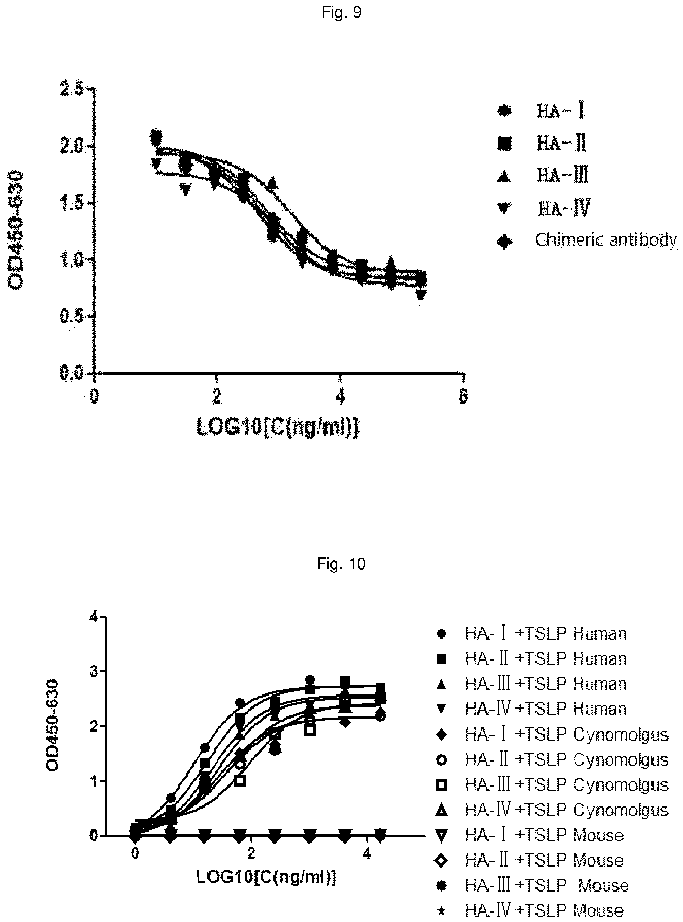

Based on the above data and as shown in Fig. 9, the 4 different humanized antibodies and the chimeric antibody screened might all inhibit the binding of TSLP to the control antibody. At the same time, among these 4 humanized antibody molecules, the IC50 value of HA-I was the lowest and its inhibitory effect was the best.

### Example 17: Cross binding experiment between humanized antibody and different species of TSLP

Human TSLP-His, mouse TSLP-His (purchased from Beijing Sino Biological Inc., product code: 51005-M08H), and monkey TSLP-His (purchased from Noboprotein Technology Co., Ltd., product code: CR62) were respectively coated with pH 9.6 carbonate buffer solution at 100 ng/well/100 µl and coated overnight at a temperature of 4°C. It was washed with 300 µl /well PBST for five times, 1% BSA-PBST was added, and it was sealed at 37°C for 1 h. Different dilution concentrations of humanized antibodies HA-I, HA-II, HA-III, and HA-IV were added, the initial highest concentrations of all 4 humanized antibodies were 16 µg/ml, and after being diluted by 4 times, each antibody was diluted for a total of 8 gradients, and incubated at 37°C for 1 h. It was washed with 300 µl/well PBST for five times, then Goat AntiHuman IgG-HRP diluted with 1% BSA-PBST 1:5000 was added and incubated at 37°C for 1 h. A TMB color kit was used for color development at 100 µl/well, it was color-developed at the room temperature for 5 min, then terminated with 2 M H₂SO₄. An ELISA plate was read at 450 nm/630 nm, and the corresponding EC50 value was calculated. Specific data was as follows.

| Clone +TSLP (Human) | HA-I | HA-II | HA-III | HA-IV |
|---|---|---|---|---|
| EC50 (ng/ml) | 9.94 | 17.17 | 27.1 | 21.34 |
| Clone +TSLP (Cynomolgus) | HA-I | HA-II | HA-III | HA-IV |
| EC50 (ng/ml) | 27.32 | 50.77 | 89.25 | 50.8 |
| Clone +TSLP (Mouse) | HA-I | HA-II | HA-III | HA-IV |
| EC50 (ng/ml) | N/A | N/A | N/A | N/A |

Based on the above data and as shown in Fig. 10, the 4 different humanized antibodies screened might all bind to the human TSLP and the cynomolgus macaque TSLP, but not to the mouse TSLP. In addition, among these 4 humanized antibody molecules, HA-I had the lowest EC50 value with the human TSLP and the cynomolgus macaque, it was indicated that its binding ability was strong. It might be used for pharmacological toxicity research and safety evaluation in an experimental animal model of the cynomolgus macaque.

### Example 18: Binding inhibition experiment between TSLP and cell surface receptor by anti-TSLP monoclonal antibody

BaF/3-TSLPR engineering cell lines were digested and counted, cells were diluted to 1×10⁶ cells/ml with a sample diluent (its ingredients included 90% IMDM, 10% FBS, and 300 µg/ml Hygromycin), and added to a 96-well plate at 100 µl/well. Humanized antibodies HA-I, HA-II, HA-III, and HA-IV and a control antibody were respectively diluted to an initial concentration of 200 µg/ml with the sample diluent, and diluted in a 3-fold gradient for a total of 10 gradients. The diluted humanized antibodies HA-I, HA-II, HA-III, and HA-IV and control antibody were respectively added into the 96-well plate containing 100 µl of the BaF/3-TSLPR cells at 50 µl/well. An antigen TSLP was diluted to 8 µg/ml with the sample diluent, and added to the above well plate containing the BaF/3-TSLPR cells, the humanized antibodies, and the control antibody at 50 µl/well. After being gently mixed uniformly, the 96-well plate was placed and incubated at 4°C for 1 h. After the incubation was completed, it was centrifuged at 3000 rpm to discard a supernatant, and a cell precipitate was collected. A pre-diluted Goat Anti-Human IgG-Fc antibody (purchased from Southern Biotech, product code: 2048-30) was added to the plate at 100 µl/well, mixed uniformly with the cell precipitate in each well, and incubated at 4°C for 1 h. After the incubation was completed, 100 µl of phosphate buffer solution (PBS) was added to each well for washing the cells, it was centrifuged at 3000 rpm to discard a supernatant, and 100 µl of PBS was added to each well for resuspending the cell precipitate. It was detected by a flow cytometry on a machine. Data was collected and the corresponding IC50 value was calculated. Specific data was as follows.

| Clone | HA-I | HA-II | HA-III | HA-IV | Control antibody |
|---|---|---|---|---|---|
| IC50(µg/ml) | 0.724 | 0.921 | 1.241 | 1.133 | 1.477 |

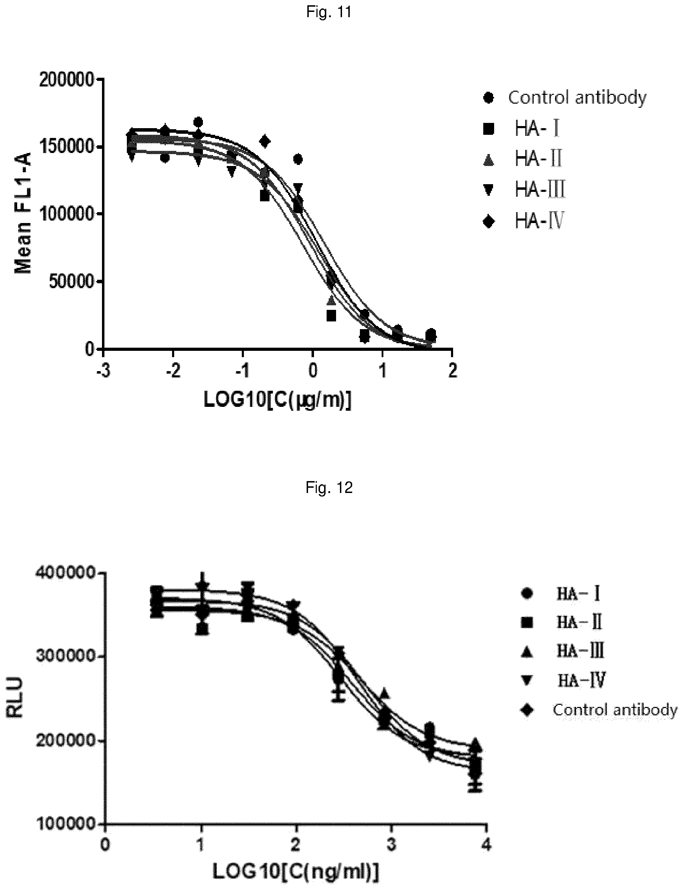

Based on the above data and as shown in Fig. 11, the 4 different humanized antibodies and the control antibody screened might compete with TSLP to bind to the cell surface receptor TSLPR. In addition, among these 4 humanized antibody molecules, the IC50 value of HA-I was the lowest and better than that of the control antibody, it was indicated that it had a good blocking effect on the binding of TSLP and its receptor at the cellular level.

### Example 19: Biological activity detection of anti-TSLP monoclonal antibody (reporter gene method)

BaF/3 cells (mouse original B cells, purchased from the Cell Resource Center of the Institute of Basic Medicine, Chinese Academy of Medical Sciences, product code: 3111 C0001 CCC000095) expressing TSLPR, IL-7Rα, and STAT5-Luc were digested and counted, cells were diluted to 1×10⁶ cells/ml with a sample diluent (its ingredients included 90% IMDM, 10% FBS, 300 µg/ml Hygromycin, 0.5 µg/ml Puromycin, and 600 µg/ml Geneticin), a TSLP-RAS-His antigen was added so that its concentration was 160 ng/ml, and after being gently mixed uniformly, cell solution was added to a 96-well plate at 50 µl/well. Humanized antibodies HA-I, HA-II, HA-III, and HA-IV and a control antibody were respectively diluted to an initial concentration of 15 µg/ml with the sample diluent, and diluted in a 3-fold gradient for a total of 8 gradients, and two replicated wells were prepared for each sample concentration. The diluted HA-I, HA-II, HA-III, and HA-IV and control antibody were respectively added into the 96-well plate containing 50 µl of cell mixed solution, and after being gently mixed uniformly, the 96-well plate was placed in a cell culture incubator and incubated for 5 h under culture conditions of 37°C and 5% CO₂. After 5 h, the 96-well plate was taken out, and it was centrifuged at 3000 rpm/min for 5 min. The solution was discarded, a Glo Lysis Buffer (purchased from Promega, product code: E2661) was added at 5 µl/well, it was lysed at a room temperature for 5 min, the cell plate was gently tapped to mix cell lysis solution uniformly, the cell lysis solution was transferred to a 384-well plate at 10 µl/well, and an equal amount of Bright-GolTM Luciferase Assay System was added, and reacted at the room temperature for 2-15 min. The fluorescence value was read under an ELISA plate, and the corresponding IC50 value was calculated. Specific data was as follows.

| Clone | HA-I | HA-II | HA-III | HA-IV | Control antibody |
|---|---|---|---|---|---|
| IC50(ng/ml) | 289.2 | 378.3 | 469.4 | 444.9 | 514.8 |

Based on the above data and as shown in Fig. 12, the 4 different humanized antibodies and the control antibody screened might bind to TSLP and compete to inhibit the binding of TSLP to the receptor complexes and exert the effects, blocking intracellular signaling pathways. The construction of engineering cell lines BaF/3-TSLPR-IL7Rα-STAT5-Luc might simulate the proliferation response of human mast cells under the action of TSLP. TSLP stimulated and upregulated the expression of an intracellular proliferation signal (STAT5-Luc) by binding to the cell surface TSLPR and IL7Ra receptors. These 4 humanized antibody molecules might effectively block the binding between TSLP and the cell surface receptors, thereby the occurrence and development of the intracellular proliferation signals were inhibited. In addition, among these 4 humanized antibody molecules, the IC50 value of HA-I was the lowest and significantly better than the control antibody, it was indicated that it might block the binding of TSLP and its receptors at the cellular level, and had the best inhibitory effect on the cell proliferation.

### Example 20: Blocking of TSLP induced release of chemokine in mDC cell by anti-TSLP monoclonal antibody

PBMC cells were resuscitated, a kit was used to select and obtain mature dendritic (DC) cells, the cell density was adjusted to 4×10⁵ cells/mL with a sample diluent (its ingredients included 90% 1640 and 10% FBS), and cell suspension was added to a 96-well plate at 50 µl/well. Humanized antibodies HA-I, HA-II, HA-III, and HA-IV and a control antibody were respectively diluted to an initial concentration of 80 ng/ml with the sample diluent, and diluted in a 3-fold gradient for a total of 8 gradients, and two replicated wells were prepared for each sample concentration. It was added to the 96-well plate containing the mature DC cells at 25 µl/well. ATSLP protein was diluted to 80 ng/ml with the sample diluent, and added to the 96-well plate containing the mature DC cells, the humanized antibodies, and the control antibody at 25 µl/well. After being gently mixed uniformly, the 96-well plate was placed in a CO₂ incubator and cultured overnight at 37°C. After about 24 h, a supernatant was taken at 50 µl/well. According to the instruction of a human TARC ELISA kit (purchased from Dakewei Biotechnology Co., Ltd., product code: 1117542), TARC detection was performed. Firstly, the diluent in the kit was used to dilute the supernatant by 3 times and mix uniformly. The diluted supernatant and standard substances were added to sample wells at 100 µl/well, and incubated at a room temperature for 2 h. After the incubation was completed, the well plate was washed with washing solution for 3 times. A Biotinylated antibody diluent was added at 100 µl/well, and incubated at the room temperature for 2 h. After the incubation was completed, the well plate was washed with washing solution for 3 times. Streptavidin-HRP working solution was added at 100 µl/well, and incubated at the room temperature for 20 min. After the incubation was completed, the well plate was washed with the washing solution for 3 times. TMB color-developing solution was added at 100 µl/well, and incubated at the room temperature in the dark for about 15 min, and the color development was terminated with termination solution at 100 µl/well. The OD value at 450 nm was read by an ELISA plate, and the corresponding IC50 value was calculated. Specific data was as follows.

| Clone | HA-I | HA-II | HA-III | HA-IV | Control antibody |
|---|---|---|---|---|---|
| IC50(ng/ml) | 100.1 | 241.1 | 313.4 | 261.3 | 584.0 |

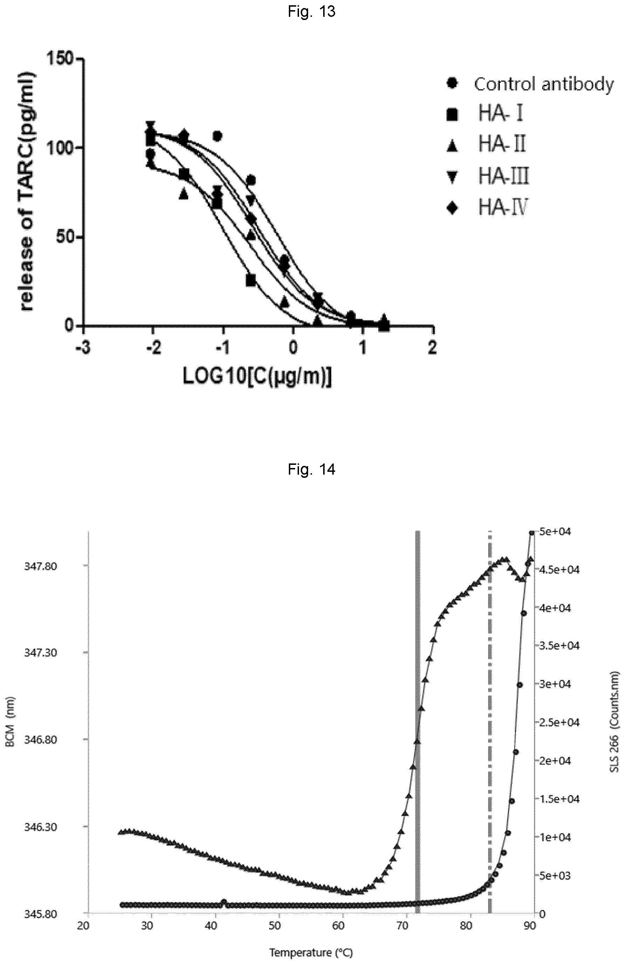

Based on the above data and as shown in Fig. 13, the 4 different humanized antibodies and the control antibody screened might all inhibit the release of the chemokine TARC from the TSLP activated mDC cells. In addition, among these 4 humanized antibody molecules, the IC50 value of HA-I is the lowest and significantly better than that of the control antibody, it was indicated that it might effectively inhibit the activation effect of TSLP on mDC at the cellular level, and the inhibitory effect was the best.

### Example 21: Evaluation of thermal stability of anti-TSLP monoclonal antibody HA-I

Anti-TSLP monoclonal antibody HA-I was transferred to a PBS buffer system by ultrafiltration, it was centrifuged at 12000 rpm and 4°C for 5 min, and the thermal stability of the anti-TSLP monoclonal antibody HA-I was evaluated with a multifunctional protein thermal stability analysis system (purchased from Unchained Labs). By monitoring changes in endogenous fluorescence of a protein with a temperature (starting from 25°C and heating at a rate of 0.3°C/min to 95°C), the changes in protein conformation were detected, to determine the protein melting temperature Tm and evaluate the stability of protein conformation. When a sample was aggregated, it might cause interference of scattered light waves and increase a scattered light signal. The colloidal stability of the protein was determined by static light scattering (represented by Tagg), and results were shown in the following table and Fig. 14.

| Sample | Tm(°C) | Tagg 266(°C) |
|---|---|---|
| 2 mg/ml anti-TSLP monoclonal antibody HA-I | 71.7 | 83.0 |

As shown in the above table and Fig. 14, the temperature of the anti-TSLP monoclonal antibody HA-I was 71.7°C, the average Tagg was 83.0 °C, and it showed the good conformation and colloidal stability.

### Example 22: Subcutaneous injection of anti-TSLP monoclonal antibody administered to cynomolgus macaque for 4-week toxicity test

The research purpose was to observe toxicity conditions of the subcutaneous injection of the anti-TSLP monoclonal antibody in cynomolgus macaques administered once a week for 4 weeks continuously.
(1) Test substance information: anti-TSLP monoclonal antibody HA-I, prepared into an injection solution (excipients included histidine, hydrochloride histidine, sorbitol, and polysorbate 80);
Reference substance information: buffer solution of anti-TSLP monoclonal antibody HA-I (ingredients included histidine, hydrochloride histidine, sorbitol, and polysorbide 80, except for the pharmaceutical ingredient anti-TSLP monoclonal antibody HA-I, other ingredients were the same as those of the injection solution) was used as a control.
(2) Provided by: Beijing Eastern Biotech Biotechnology Co., Ltd.
(3) Experimental animal:
Species and strain: cynomolgus macaques;
Age: 2.5-5 years old;
Weight: 2-5 kg;
Gender: female and male; and
Animal quantity: 8, 4 females and 4 males each.

(4) Grouping and administration

| Group | Reference substance/test substance | Dose (mg/kg) | Capacity (mUkg) | Animal quantity (animals/gender) |
|---|---|---|---|---|
| 1 | Buffer solution of anti-TSLP monoclonal antibody HA-I | 0 | 2.00 | 1 female and 1 male each |
| 2 | Injection solution of anti-TSLP monoclonal antibody HA-I | 30 | 0.20 | 1 female and 1 male each |
| 3 | Injection solution of anti-TSLP monoclonal antibody HA-I | 100 | 0.67 | 1 female and 1 male each |
| 4 | Injection solution of anti-TSLP monoclonal antibody HA-I | 300 | 2.00 | 1 female and 1 male each |

Subcutaneous injection administration, the first administration was recorded as D1, afterwards it was administered once a week for 4 weeks continuously, and there was a total of 5 administrations.

In this experiment, the following parameters were evaluated: clinical symptom observation, body weight, food intake, body temperature, detection of accompanied safety and pharmacological indicators, electrocardiogram, blood pressure, ophthalmic examination, clinical pathological examination (blood cell count, coagulation function, blood biochemical analysis, and urine analysis), immunological indicators (immune cell phenotype, cytokines, immunoglobulins, and complements), anti-drug antibodies, and toxicokinetics.

Experimental result: during the experiment, the clinical observation, body weight, body temperature, electrocardiogram parameters, blood cell count, blood biochemistry, urine examination, T lymphocyte subsets, and gross anatomical observation of animals in Groups 2-4 showed no significant or regular abnormal changes in all indicators.

In addition, the female animals in the 300 mg/kg dose group showed an increase in FIB before the second administration (D8) and the day after the third administration (D16), and returned to normal on the day after the last administration (D30). No abnormalities were observed in the other animals. 1-2 hours after the first administration (D1), IL-6 was increased in the male animals in the 30 mg/kg dose group and IL-10 was increased in the female animals in the 300 mg/kg dose group, and all were recovered on the next day. On the day after the first administration (D2), IL-6 was increased in both male and female animals in the 300 mg/kg dose group, and returned to normal before the administration on D8. Before the second administration (D8), IL-10 was also increased in the female animals in the 300 mg/kg dose group, and returned to normal on D29, no significant abnormal changes were observed in the rest.

During the experiment, no ADA was detected in any groups of the animals.

Toxicokinetic results showed that the anti-TSLP monoclonal antibody HA-I was subcutaneously injected into the cynomolgus macaques at doses of 30, 100, and 300 mg/kg respectively. After the first and fourth administrations, the blood drug concentration and exposure were increased with the increase of the dose, and no significant gender differences were observed in the main toxicokinetic parameters. After being administered once a week for 4 weeks continuously, EB070 was slightly accumulated in the cynomolgus macaques (the accumulation factor was 1.49-2.71).

### Example 23: Subcutaneous injection of anti-TSLP monoclonal antibody administered to cynomolgus monkey for 13-week toxicity test

On the basis of Example 22, the purpose of this experimental research was to evaluate toxic reactions and toxicokinetics conditions of the anti-TSLP monoclonal antibody administered once a week and repeatedly subcutaneous-injected in cynomolgus macaques for 13 weeks, as well as recovery conditions of toxicity after 6 weeks of drug discontinuance, by injecting the anti-TSLP monoclonal antibody.
(1) Test substance information: anti-TSLP monoclonal antibody HA-I, prepared into an injection solution (excipients included histidine, hydrochloride histidine, sorbitol, and polysorbate 80);
Reference substance information: buffer solution of anti-TSLP monoclonal antibody HA-I (ingredients included histidine, hydrochloride histidine, sorbitol, and polysorbide 80, except for the pharmaceutical ingredient anti-TSLP monoclonal antibody HA-I, other ingredients were the same as those of the injection solution) was used as a control.
(2) Provided by: Beijing Eastern Biotech Biotechnology Co., Ltd.
(3) Experimental animal:
Species and strain: cynomolgus macaques;
Age: 3-5 years old;
Weight: 2-5 kg;
Gender: female, and male; and
Animal quantity: 40, 20 females and 20 males each.

(4) Grouping and administration

| Group | Reference substance/test substance | Dose (mg/kg) | Concentration (mg/mL) | Capacity (mL/kg) | Animal quantity (animals/gender) |
|---|---|---|---|---|---|
| 1 | Buffer solution of anti-TSLP monoclonal antibody HA-I | 0 | 0 | 2.00 | 3 females and 2 males respectively |
| 2 | Injection solution of anti-TSLP monoclonal antibody HA-I | 10 | 150 | 0.067 | 3 females and 2 males respectively |
| 3 | Injection solution of anti-TSLP monoclonal antibody HA-I | 60 | 150 | 0.40 | 3 females and 2 males respectively |
| 4 | Injection solution of anti-TSLP monoclonal antibody HA-I | 300 | 150 | 2.00 | 3 females and 2 males respectively |

Subcutaneous injection administration, the first administration was recorded as D1, afterwards it was administered once a week for 13 weeks continuously, and there was a total of 14 administrations; and in this experiment, the following parameters were evaluated: clinical symptom observation, body weight, food intake, body temperature, detection of accompanied safety and pharmacological indicators, electrocardiogram, blood pressure, ophthalmic examination, clinical pathological examination (blood cell count, coagulation function, blood biochemical analysis, and urine analysis), immunological indicators (immune cell phenotype, cytokines, immunoglobulins, and complements), anti-drug antibodies, and toxicokinetics.

Preliminary experimental results showed that: after repeated subcutaneous injection of the anti-TSLP monoclonal antibody HA-I, there were no significant abnormalities observed in the clinical observation, body weight, body temperature, ophthalmic examination, and gross autopsy of the cynomolgus macaques.

The present invention is not limited to preferred implementation modes above, and any other forms of products may be derived from the inspiration of the present invention by any persons. However, regardless of any changes in its shape or structure, any technical schemes that are the same or similar to the present invention shall fall within the scope of protection of the present invention.

## Claims

1. An anti-TSLP monoclonal antibody or an antigen-binding fragment thereof, comprising a heavy chain variable region and a light chain variable region, wherein the heavy chain variable region comprises three heavy chain complementarity-determining regions represented by HCDR1, HCDR2, and HCDR3 respectively, the light chain variable region comprises three light chain complementarity-determining regions represented by LCDR1, LCDR2, and LCDR3 respectively, and the monoclonal antibody or the antigen-binding fragment thereof is selected from any one of the following:
A-I: the heavy chain complementarity-determining region HCDR1 comprises an amino acid sequence as shown in SEQ ID NO: 1, the heavy chain complementarity-determining region HCDR2 comprises an amino acid sequence as shown in SEQ ID NO: 2, the heavy chain complementarity-determining region HCDR3 comprises an amino acid sequence as shown in SEQ ID NO: 3, the light chain complementarity-determining region LCDR1 comprises an amino acid sequence as shown in SEQ ID NO: 4, the light chain complementarity-determining region LCDR2 comprises an amino acid sequence as shown in SEQ ID NO: 5, and the light chain complementarity-determining region LCDR3 comprises an amino acid sequence as shown in SEQ ID NO: 6;
A-II: the heavy chain complementarity-determining region HCDR1 comprises an amino acid sequence as shown in SEQ ID NO: 7, the heavy chain complementarity-determining region HCDR2 comprises an amino acid sequence as shown in SEQ ID NO: 8, the heavy chain complementarity-determining region HCDR3 comprises an amino acid sequence as shown in SEQ ID NO: 9, the light chain complementarity-determining region LCDR1 comprises an amino acid sequence as shown in SEQ ID NO: 4, the light chain complementarity-determining region LCDR2 comprises an amino acid sequence as shown in SEQ ID NO: 10, and the light chain complementarity-determining region LCDR3 comprises an amino acid sequence as shown in SEQ ID NO: 6;
A-III: the heavy chaincomplementarity-determining region HCDR1 comprises an amino acid sequence as shown in SEQ ID NO: 1, the heavy chain complementarity-determining region HCDR2 comprises an amino acid sequence as shown in SEQ ID NO: 11, the heavy chain complementarity-determining region HCDR3 comprises an amino acid sequence as shown in SEQ ID NO: 3, the light chain complementarity-determining region LCDR1 comprises an amino acid sequence as shown in SEQ ID NO: 12, the light chain complementarity-determining region LCDR2 comprises an amino acid sequence as shown in SEQ ID NO: 13, and the light chain complementarity-determining region LCDR3 comprises an amino acid sequence as shown in SEQ ID NO: 14; and
A-IV: the heavy chaincomplementarity-determining region HCDR1 comprises an amino acid sequence as shown in SEQ ID NO: 1, the heavy chain complementarity-determining region HCDR2 comprises an amino acid sequence as shown in SEQ ID NO: 11, the heavy chain complementarity-determining region HCDR3 comprises an amino acid sequence as shown in SEQ ID NO: 3, the light chain complementarity-determining region LCDR1 comprises an amino acid sequence as shown in SEQ ID NO: 12, the light chain complementarity-determining region LCDR2 comprises an amino acid sequence as shown in SEQ ID NO: 15, and the light chain complementarity-determining region LCDR3 comprises an amino acid sequence as shown in SEQ ID NO: 16.

2. The anti-TSLP monoclonal antibody or the antigen-binding fragment thereof according to claim 1, wherein the anti-TSLP monoclonal antibody or the antigen-binding fragment thereof is a murine antibody molecule, and the murine antibody molecule is selected from any one of the following:
MA-I: the heavy chain variable region comprises an amino acid sequence as shown in SEQ ID NO: 17, and the light chain variable region comprises an amino acid sequence as shown in SEQ ID NO: 18;
MA-II: the heavy chain variable region comprises an amino acid sequence as shown in SEQ ID NO: 19, and the light chain variable region comprises an amino acid sequence as shown in SEQ ID NO: 20;
MA-III: the heavy chain variable region comprises an amino acid sequence as shown in SEQ ID NO: 21, and the light chain variable region comprises an amino acid sequence as shown in SEQ ID NO: 22; and
MA-IV: the heavy chain variable region comprises an amino acid sequence as shown in SEQ ID NO: 23, and the light chain variable region comprises an amino acid sequence as shown in SEQ ID NO: 24;
preferably, the murine antibody molecule is MA-I.

3. The anti-TSLP monoclonal antibody or the antigen-binding fragment thereof according to claim 2, wherein the murine antibody molecule further comprises a murine antibody heavy chain constant region and a murine antibody light chain constant region, the murine antibody heavy chain constant region is selected from a heavy chain constant region of a mouse IgG1-type, IgG2a-type, IgG2b-type or IgG3-type, wherein the amino acid sequence of the IgG1-type heavy chain constant region is shown in SEQ ID NO: 26, the amino acid sequence of the IgG2a-type heavy chain constant region is shown in SEQ ID NO: 27, the amino acid sequence of the IgG2b-type heavy chain constant region is shown in SEQ ID NO: 28, and the amino acid sequence of the IgG3-type heavy chain constant region is shown in SEQ ID NO: 29; the murine antibody light chain constant region is a mouse Cₖ-type light chain constant region, and an amino acid sequence thereof is shown in SEQ ID NO: 25; and
preferably, the murine antibody molecule comprises the mouse IgG1-type heavy chain constant region and the mouse Cₖ-type light chain constant region.

4. The anti-TSLP monoclonal antibody or the antigen-binding fragment thereof according to claim 2, wherein the monoclonal antibody or the antigen-binding fragment thereof is a chimeric antibody molecule, the chimeric antibody molecule comprises a heavy chain variable region of the murine antibody molecule, a light chain variable region of the murine antibody molecule, and a humanized antibody constant region.

5. The anti-TSLP monoclonal antibody or the antigen-binding fragment thereof according to claim 1, wherein the monoclonal antibody or the antigen-binding fragment thereof is a humanized antibody molecule, and the humanized antibody molecule is selected from any one of the following:
HA-I: the heavy chain variable region comprises an amino acid sequence as shown in SEQ ID NO: 34, and the light chain variable region comprises an amino acid sequence as shown in SEQ ID NO: 35;
HA-II: the heavy chain variable region comprises an amino acid sequence as shown in SEQ ID NO: 34, and the light chain variable region comprises an amino acid sequence as shown in SEQ ID NO: 36;
HA-III: the heavy chain variable region comprises an amino acid sequence as shown in SEQ ID NO: 37, and the light chain variable region comprises an amino acid sequence as shown in SEQ ID NO: 38; and
HA-IV: the heavy chain variable region comprises an amino acid sequence as shown in SEQ ID NO: 37, and the light chain variable region comprises an amino acid sequence as shown in SEQ ID NO: 36;
preferably, the humanized antibody molecule is HA-I.

6. The anti-TSLP monoclonal antibody or the antigen-binding fragment thereof according to claim5, wherein the humanized antibody molecule further comprises a humanized antibody constant region.

7. The anti-TSLP monoclonal antibody or the antigen-binding fragment thereof according to claim 5 or 6, wherein the humanized antibody molecule is a full-length antibody or an antibody fragment, and the humanized antibody molecule comprises one or a combination of Fab, F(ab)2, Fv, or ScFv.

8. The anti-TSLP monoclonal antibody or the antigen-binding fragment thereof according to claim 4 or 6, wherein the humanized antibody constant region comprises a humanized antibody heavy chain constant region and a humanized antibody light chain constant region, the humanized antibody heavy chain constant region is selected from a heavy chain constant region of a human IgG1-type, IgG2-type, or IgG4-type, the amino acid sequence of the IgG1-type heavy chain constant region is shown in SEQ ID NO: 30, the amino acid sequence of the IgG2-type heavy chain constant region is shown in SEQ ID NO: 31, the amino acid sequence of the IgG4-type heavy chain constant region is shown in SEQ ID NO: 32, the humanized antibody light chain constant region is a human Cₖ-type light chain constant region, and an amino acid sequence thereof is shown in SEQ ID NO: 33; and
preferably, the humanized antibody constant region comprises the human IgG1-type heavy chain constant region and the human Cₖ-type light chain constant region.

9. A protein, comprising the anti-TSLP monoclonal antibody or the antigen-binding fragment thereof according to any one of claims 1-8.

10. A polynucleotide molecule, wherein the polynucleotide molecule encodes the anti-TSLP monoclonal antibody or the antigen-binding fragment thereof according to any one of claims 1-8.

11. A recombinant DNA expression vector, wherein the recombinant DNA expression vector comprises the polynucleotide molecule according to claim 10.

12. A host cell transfected with the recombinant DNA expression vector according to claim 11, wherein the host cell comprises a prokaryotic cell, a yeast cell, an insect cell, or a mammalian cell; and
preferably, the host cell is the mammalian cell, and the mammalian cell is a HEK293 cell, a CHO cell, or a NS0 cell.

13. A drug, wherein the drug comprises the anti-TSLP monoclonal antibody or the antigen-binding fragment thereof according to any one of claims 1-8.

14. Use of the anti-TSLP monoclonal antibody or the antigen-binding fragment thereof according to any one of claims 1-8 in preparation of a drug for treating an immunological disease or a cancer;
preferably, the immunological disease comprises asthma, chronic obstructive pulmonary disease, chronic eosinophilic pneumonia, idiopathic pulmonary fibrosis, and allergic dermatitis; the asthma comprises severe asthma, eosinophilic or non-eosinophilic asthma, and low eosinophilic asthma; and
preferably, the cancer comprises pancreatic cancer, non-small cell lung cancer, melanoma, prostate cancer, kidney cancer, colorectal cancer, or breast cancer.

15. A method for treating or preventing a TSLP mediated disease, wherein the method comprises administering a therapeutically effective amount of the anti-TSLP monoclonal antibody according to any one of claims 1-8 to an individual in need, and the disease comprises an immunological disease or a cancer;
the immunological disease comprises asthma, chronic obstructive pulmonary disease, chronic eosinophilic pneumonia, idiopathic pulmonary fibrosis, and allergic dermatitis; the asthma comprises severe asthma, eosinophilic or non-eosinophilic asthma, and low eosinophilic asthma; and
the cancer comprises pancreatic cancer, non-small cell lung cancer, melanoma, prostate cancer, kidney cancer, colorectal cancer, or breast cancer.
